# EUROPEAN PATENT APPLICATION

(11) **EP 0 893 142 A1**
(43) Date of publication of application: **27.01.1999**
(21) Application number: 96909351.7
(22) Date of filing: 10.04.1996
(51) Int. Cl.: A62B 18/02

(54) **MASK FOR TREATING COLD**

(71) Applicant: Yasuda, Saburo, Hyogo 662 (JP)
(72) Inventor: Yasuda, Saburo, Hyogo 662 (JP)
(74) Representative: Brehm, Hans-Peter, Dr., Dipl.-Chem.
(86) International application number: JP9601007
(87) International publication number: WO9632160

(57) **Abstract**

Object: to treat cold and pollinosis.
Constitution: a mask that enables slow release of drugs, which is composed of a liquid-impermeable and gas-permeable film and drugs, wherein the said drugs deactivate influenza viruses on the upper respiratory mucous membrane and inhibit the absorption of pollen antigen onto the mucous membrane.
Effect: the fever is lowered several hours after wearing of the mask. Also the snivels are suspended during wearing of the mask.

## Description

### [Technical Field]

The invention relates to the mask for the therapy of common cold and pollinosis, wherefrom the drugs are slowly released and then inhaled.

### [Background]

The present inventor disclosed in Japanese Patent 60-240330 that common cold could be cured with the inhaling of ethanol vapor, etc. After that, the present inventor found that the continuos inhaling of ethanol vapor, etc. is more effective for the therapy.

### [Disclosure of Invention]

This invention relates to the mask comprising the drugs such as ethanol (Japan Pharmacopoeia grade), allyl sulfide, *l*-menthol, radish juice, and etc., are wrapped in a liquid-impermeable and gas- permeable film, for the purpose of slow-release and inhaling of drugs.

The released drugs from the mask anesthetize upper respiratory mucous membrane on which viruses are proliferated and deactivate the viruses. Another effect is that the released drugs inhibit the adsorption of pollen antigens onto the mucous membrane, thus relieving the symptoms caused from pollinosis.

### [Brief Description of Drawings]

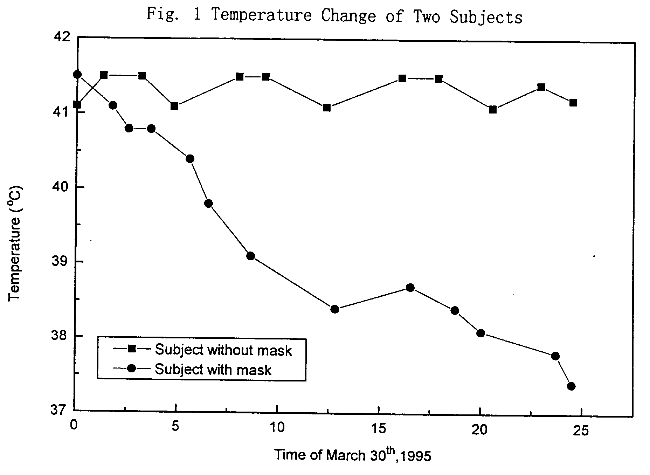

- Fig. 1: The figure represents the temperature change of two volunteer subjects, one wearing the mask and another not wearing the mask.
● denotes the subject wearing the mask
■ denotes the subject not wearing the mask.

### [Best Mode for Carrying out the Invention]

The drugs such as ethanol (Japan Pharmacopoeia grade) was wrapped into a liquid-impermeable and gas-permeable film; and the wrapped film thus obtained was equipped into a mask. The masks thus prepared were applied to the two volunteers, the details of volunteer study are described below.
- Time:: March 30^{th} 1995 to March 31^{st} 1995
- Place:: Yasui Elementary School, Hanshin Earthquake Shelter No. 5
- Subject:: A elder sister (age 15) wearing the mask
A younger sister (age 13) not wearing the mask
- Treatments:: Influenza (probably B type) was infected simultaneously to the sisters from their father, and their temperatures rose up to 41°C.
The younger sister (without mask) was in bed all days on 30^{th}. In the morning of 31^{st}, the temperature got down to 37°C. The elder sister (with mask) went to school on 30^{th}, the temperature got down to 37°C when she came back home from school.
The temperature changes of two subjects are shown in Fig. 1

### [Industrial Applicability]

This invention relates to the therapy of common cold and pollinosis.

## Claims

1. Mask for the therapy of common-cold and pollinosis, from which drugs that anesthetize upper respiratory mucous membrane and deactivate viruses on the membrane are slowly released.
